(19) Europäisches Patentamt
European Patent Office
Office européen des brevets

(11) **EP 1 880 000 B1**

(12) **FASCICULE DE BREVET EUROPEEN**

(45) Date de publication et mention
de la délivrance du brevet:
**03.09.2014 Bulletin 2014/36**

(51) Int Cl.:
*A23L 1/30* (2006.01)    *C12N 1/16* (2006.01)
*A23L 1/29* (2006.01)    *A61K 8/23* (2006.01)
*A61K 8/64* (2006.01)    *A61K 8/99* (2006.01)
*A61Q 17/00* (2006.01)

(21) Numéro de dépôt: **06743624.6**

(22) Date de dépôt: **30.03.2006**

(86) Numéro de dépôt international:
**PCT/FR2006/000693**

(87) Numéro de publication internationale:
**WO 2006/103350 (05.10.2006 Gazette 2006/40)**

(54) **PREPARATIONS DE LEVURES A PROPRIETES ANTI-OXYDANTES AMELIOREES ET LEURS APPLICATIONS**

HEFEPRÄPARATIONEN MIT VERBESSERTEN ANTIOXIDATIVEN EIGENSCHAFTEN UND VERWENDUNGEN DAVON

YEAST PREPARATIONS WITH IMPROVED ANTIOXIDANT PROPERTIES AND USES THEREOF

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **30.03.2005 FR 0503086**

(43) Date de publication de la demande:
**23.01.2008 Bulletin 2008/04**

(73) Titulaire: **Danstar Ferment AG**
**6300 Zug (CH)**

(72) Inventeurs:
• **DEGRE, Richard**
**Québec, Québec J3V 3X3 (CA)**
• **BAULEZ, Matthieu**
**F-31170 Tournefeuille (FR)**
• **ZHIGEN, Zhang**
**Montreal, Québec H4V 2G3 (CA)**
• **FORBES, Wardrop**
**Quebec, Québec H9R 3B7 (CA)**

(74) Mandataire: **Peaucelle, Chantal et al**
**Cabinet Armengaud Aîné**
**3, Avenue Bugeaud**
**75116 Paris (FR)**

(56) Documents cités:
**EP-A- 0 797 929        WO-A-00/28977**
**WO-A-98/37172        US-B1- 6 337 320**

• ANONYME: "SÉLÉNIUM Forté - Une réponse complète pour préserver votre capital jeunesse" ARTICLE INTERNET, [Online] 1 juillet 2004 (2004-07-01), XP002354037 Extrait de l'Internet: URL:http://web.archive.org/web/20040701092 323/http://www.ponroy.com/fr/s10_boutique_ nutri/s10p02_zoomproduitnutri.php?id_produ it=271> [extrait le 2005-11-14]
• SUHAJDA ET AL: "Preparation of selenium yeasts I. Preparation of selenium-enriched Saccharomyces cerevisiae" JOURNAL OF TRACE ELEMENTS IN MEDICINE AND BIOLOGY, FISCHER, NEW YORK, NY, US, vol. 14, no. 1, avril 2000 (2000-04), pages 43-47, XP005034097 ISSN: 0946-672X
• PATENT ABSTRACTS OF JAPAN vol. 2003, no. 12, 5 décembre 2003 (2003-12-05) & JP 2004 180509 A (KANEGAFUCHI CHEM IND CO LTD), 2 juillet 2004 (2004-07-02)
• ALFAFARA C G ET AL: "EFFECT OF AMINO ACIDS ON GLUTATHIONE PRODUCTION BY SACCHAROMYCES CEREVISIAE" APPLIED MICROBIOLOGY AND BIOTECHNOLOGY, SPRINGER VERLAG, BERLIN, DE, vol. 36, no. 4, 1992, pages 538-540, XP008012042 ISSN: 0175-7598

- BANSAL M P ET AL: "Growth characteristics and selenium status changes of yeast cells with inorganic and organic selenium supplementation: Selenium, a chemopreventive agent" JOURNAL OF MEDICINAL FOOD, vol. 5, no. 2, juillet 2002 (2002-07), pages 85-90, XP009056753 ISSN: 1096-620X
- EL-BAYOUMY KARAM ET AL: "Influence of selenium-enriched yeast supplementation on biomarkers of oxidative damage and hormone status in healthy adult males: A clinical pilot study." CANCER EPIDEMIOLOGY BIOMARKERS AND PREVENTION, vol. 11, no. 11, novembre 2002 (2002-11), pages 1459-1465, XP002354039 ISSN: 1055-9965
- MEISTER A: "GLUTATHIONE METABOLISM AND ITS SELECTIVE MODIFICATION" JOURNAL OF BIOLOGICAL CHEMISTRY, vol. 263, no. 33, 1988, pages 17205-17208, XP002354040 ISSN: 0021-9258

**Description**

L'invention a pour objet des préparations de levures présentant des propriétés anti-oxydantes améliorées et leurs applications comme additifs anti-oxydants.

On sait que les levures riches en sélénium, c'est-à-dire titrant plus de 500 ppm environ de sélénium, et avantageusement de l'ordre de 2000 ppm de sélénium, sont utilisées en routine comme compléments alimentaires en nutrition humaine et animale.

Anonymous (2004) décrit une composition nommée "Sélénium forte" qui contient des antioxydants tels que le sélénium et le glutathion.

Dans le cadre de leur recherche de moyens pour améliorer les propriétés anti-oxydantes de telles levures, les inventeurs ont étudié les effets d'ajout de diverses formes de sélénium et/ou de glutathion aux levures. Ils ont ainsi constaté que, de manière inattendue, les propriétés anti-oxydantes de levures pouvaient être fortement améliorées par un enrichissement en sélénium et en glutathion produit en cours de fermentation par les levures, ou ajouté après fermentation.

L'invention a donc pour but de fournir des préparations de levures dotées de propriétés anti-oxydantes améliorées.

Au sens le plus large, l'invention porte sur des objets tels que définis dans les revendications.

Elle vise également leurs applications comme additifs anti-oxydants, en particulier comme compléments alimentaires en nutrition humaine et comme additifs pour l'alimentation animale.

Les préparations de levures de l'invention, présentant des propriétés anti-oxydantes améliorées, sont caractérisées en ce qu'elles renferment des levures enrichies concomitamment en glutathion et en sélénium.

Il est en effet apparu que la présence concomitante de glutathion et de sélénium dans une levure conduisait, de manière inattendue, à un effet synergique et permettait d'obtenir des propriétés anti-oxydantes supérieures à celles observées avec d'autres modes d'ajouts du glutathion et du sélénium.

Par "levure enrichie en glutathion et en sélénium", on entend une levure titrant au moins 15mg de glutathion par gramme de matières sèches et au moins 500 ppm en sélénium.

De manière préférée, lesdites levures sont des levures du genre *Saccharomyces,* et notamment de l'espèce cerevisiae.

Il s'agit notamment de levures de panification.

Les méthodes de production de levure enrichie en sélénium sont bien connues de l'homme de l'art (voir notamment US 4 530 846). Les méthodes de production de levure au glutathion sont également largement décrites (voir notamment JP 52156994 et JP 61132282). Les levures enrichies concomitamment en sélénium et glutathion peuvent être obtenues en combinant les 2 types de méthodes connues.

Compte tenu de leurs propriétés, les préparations de levures définies ci-dessus présentent un grand intérêt, de manière générale, comme additifs anti-oxydants.

L'invention vise en particulier leur utilisation comme compléments alimentaires pour l'homme ou l'animal, ainsi que les aliments ou nutriments ou alicaments qui les renferment.

On citera entre autres des biscuits et pains enrichis par ces préparations, ou encore des produits laitiers comme les yaourts ainsi que des aliments pour animaux.

Les préparations de l'invention sont également avantageuses dans le domaine cosmétique, en conférant des propriétés anti-vieillissement aux compositions cosmétiques, ou comme principes actifs de médicaments pour prévenir ou traiter les pathologies associées à la formation de radicaux libres en excès.

Dans ces diverses applications, les préparations de l'invention sont utilisées notamment sous forme de poudres ou encore de lyophilisats. De manière générale, elles se présentent sous des formes incorporables dans les préparations alimentaires, pharmaceutiques (comprimés, gélules,...) ou cosmétiques (crèmes, onguents, ...).

Pour des applications en nutrition humaine, on a avantageusement recours à des doses de 75 à 100 μg/j en Se. En nutrition animale, des concentrations maximales de 0,5 ppm de Se total dans l'aliment complet apparaissent appropriées. Ces utilisations peuvent être de longue durée.

Est également décrit un procédé d'obtention de préparations de levures telles que définies ci-dessus, caractérisé par la mise en oeuvre de levures titrant environ 15 mg de glutathion/g de matières sèches et contenant des traces de sélénium, et de levures titrant environ au moins 500 ppm de sélénium avantageusement de l'ordre de 2000 ppm de sélénium. Avantageusement, on met en oeuvre des levures titrant à la fois 15 mg environ de glutathion/g de matières sèches et au moins 500 ppm environ, avantageusement de l'ordre de 2000 ppm, de sélénium.

D'autres caractéristiques et avantages de l'invention sont donnés dans les exemples qui suivent.

Exemple 1 : Etude comparative des propriétés anti-oxydantes d'échantillons de levures

On rapporte tout d'abord les résultats relatifs aux propriétés anti-oxydantes des échantillons étudiés mesurées selon les 3 méthodes suivantes :

- la méthode FOLIN : Cette méthode permet de mesurer par colorimétrie le pouvoir antioxydant de nombreux types d'antioxydants susceptibles d'être contenus dans les différents échantillons. Ces antioxydants peuvent être des acides aminés facilement oxydables (tryptophane, tyrosine), des composés de sélénium, et le glutathion. Cette méthode est relativement ancienne, mais est encore couramment utilisée pour mesurer les effets d'antioxydants dans les produits alimentaires et les boissons [Methods of Enzymology, Vol.335 p.103-114 (2001)] ;
- la méthode FRAP : La méthode FRAP (Ferric Reducing Antioxidant Power) mise au point par Benzie (Anal.Biochem. 1996 Jul.15, 239(1) :70-6) est également largement reconnue pour les mesures de pouvoir antioxydant. Cette méthode plus sélective utilise un standard différent de celui utilisé par la méthode FOLIN (tocophérol). De ce fait, les valeurs obtenues par FRAP sont plus basses que celles obtenues par FOLIN ;
- la méthode LAG-TIME (temps de retard)/LAG-TIME lié.

La mesure de l'oxydation des lipoprotéines de faible densité (Lower Density Lipoproteins ou LDL en abrégé) permet de comparer différents anti-oxydants lors de maladies cardiovasculaires (Free Radic.Biol.Med. 2000 Jun.15, 28(12):1815-26). La méthode standard a été utilisée à pH et température physiologiques [Methods of Enzymology Vol. 335 p. 103-114 (2001)]. Elle permet de mesurer le temps nécessaire aux antioxydants ajoutés pour s'oxyder. Ceci correspond au moment précédant le processus rapide d'oxydation des lipoprotéines (temps de retard) lorsque l'absorbance augmente de façon importante. L'effet de l'antioxydant présent est d'autant plus fort que le temps de retard est long (en secondes). L'analyse cinétique a été réalisée à 234 nm. Les différents échantillons ont été apportés à la même concentration de 1 $\mu$M.

On rapporte ci-après les résultats obtenus avec les 9 échantillons de levure du tableau 1 suivant :

**Tableau 1**

| Description des échantillons | | | | | |
|---|---|---|---|---|---|
| N° Ech. | Dénomination | Se (ppm) | | GSH (mg/g) | |
| | | Inorganique | Organique | Inorganique | Organique |
| 1 | LBI2133 | 0 | 0 | N | N |
| 2 | LBI2133 | 2000 | 0 | N | N |
| 3 | LBI2133 | 0 | 0 | O | N |
| 4 | LBI2133 | 2000 | 0 | O | N |
| 5 | Lalmin Se-2000 | 0 | 2000 | N | N |
| 6 | Lalmin Se-2000 | 0 | 2000 | O | N |
| 7 | Lalmin Antiox | 0 | 2000 | N | O |
| 8 | FSR* | 0 | 0 | N | O |
| 9 | FSR + Se salt | 2000 | 0 | N | O |
| * Fermaid Super Relax | | | | | |

Par « sélénium inorganique », on entend du sélénium exogène, sous forme de sels, c-à-d ajouté à la levure, alors que l'expression « sélénium organique » désigne du sélénium ajouté au milieu de fermentation et incorporé par la levure durant la fermentation.

Par « GSH inorganique », on entend du GSH exogène, c-à-d ajouté à la levure, alors que l'expression « GSH organique » désigne du GSH endogène, c-à-d produit par la levure même durant la fermentation.

## a- Méthode FOLIN

Les mesures des concentrations en phénol sur les différents échantillons de levures ont donné les résultats rapportés dans le tableau 2 suivant.

**Tableau 2**
**Concentration en Phénol des différents échantillons de levure**

| N° Ech. | 1 | 2 | 3 | 4 | 5 | 6 | 7 | 8 | 9 |
|---|---|---|---|---|---|---|---|---|---|
| [Phénol] (umol/g) | 8,44 | 8,27 | 13,87 | 14,29 | 8,40 | 15,12 | 32,27 | 16,36 | 14,54 |
| | 8,35 | 8,40 | 14,45 | 14,95 | 8,75 | 14,54 | 32,92 | 14,70 | 15,04 |
| | 8,31 | 8,09 | 14,70 | 14,87 | 8,75 | 14,45 | 32,60 | 15,70 | 15,12 |
| Moyenne %STD | 8,37 | 8,25 | 14,34 | 14,70 | 8,63 | 14,70 | 32,60 | 15,59 | 14,90 |
| | 1% | 2% | 3% | 2% | 2% | 2% | 1% | 5% | 2% |

Dans le tableau 3, on indique les calculs d'analyse statistique des concentrations en phénol où « oui » correspond à une différence statistiquement significative entre les groupes (P≤0,05) et « non » à une différence non statistiquement significative entre les groupes (P>0,05).

**Tableau 3**
**Analyse statistique des concentrations en Phénol**

| N°Ech. | 1 | 2 | 3 | 4 | 5 | 6 | 7 | 8 |
|---|---|---|---|---|---|---|---|---|
| 2 | non | / | / | / | / | / | / | / |
| 3 | oui | oui | / | / | / | / | / | / |
| 4 | oui | oui | non | / | / | / | / | / |
| 5 | non | non | oui | oui | / | / | / | / |
| 6 | oui | oui | non | non | oui | / | / | / |
| 7 | oui | oui | oui | oui | oui | oui | / | / |
| 8 | oui | oui | oui | non | oui | non | oui | / |
| 9 | oui | oui | non | non | oui | non | oui | non |

"Oui" : différence statistiquement significative entre les groupes (P≤0,05).
"Non" : différence non statistiquement significative entre les groupes (P>0,05).

Les résultats obtenus montrent que l'addition :

- du GSH inorganique (1 vs. 3) ou organique (1 vs. 8) à une levure classique permet d'augmenter significativement le pouvoir antioxydant (8,37 / 14,34 / 15,59).
- du GSH inorganique à une levure riche en Se organique (5 vs. 6) permet d'augmenter significativement le pouvoir antioxydant (8,63 vs. 14,70).
- du GSH inorganique à une levure riche en Se inorganique (2 vs. 4) permet d'augmenter significativement le pouvoir antioxydant (8,25 vs. 14,70).
- du Se inorganique (1 vs. 2) ou organique (1 vs. 5) à une levure classique ne permet pas d'augmenter le pouvoir antioxydant (8,37 / 8,25 / 8,63).
- du Se inorganique à une levure riche en GSH organique (8 vs. 9) ne permet pas d'augmenter le pouvoir antioxydant (15,59 / 14,90).
- du Se inorganique à une levure riche en GSH inorganique (3 vs. 4) ne permet pas d'augmenter le pouvoir antioxydant (14,34 / 14,70).

De manière surprenante, l'apport simultané de Se et de GSH organique (Lalmin Antiox : échantillon 7) permet une augmentation très significative du pouvoir antioxydant vs. les autres traitements (32,60).

Cet échantillon de levure Lalmin Antiox (éch.7) présente un pouvoir antioxydant qui est très significativement supérieur à celui des levures soumises à des traitements différents.

Ces résultats démontrent que l'apport simultané par une même levure de Se organique et de GSH organique ajoutés en cours de fermentation présente un intérêt manifeste pour accroître le potentiel antioxydant.

**b- <u>Méthode FRAP</u>**

Les résultats obtenus avec les différents échantillons sont donnés dans le tableau 4 ci-après.

**Tableau 4**
**Résultats FRAP des différents échantillons**

| N°Ech. | 1 | 2 | 3 | 4 | 5 | 6 | 7 | 8 | 9 |
|---|---|---|---|---|---|---|---|---|---|
| FRAP ($\mu$mol/g) | 3,25 | 3,46 | 3,61 | 3,57 | 3,06 | 4,14 | 7,77 | 4,14 | 4,41 |
| | 3,46 | 3,42 | 3,42 | 3,48 | 2,96 | 4,14 | 7,70 | 4,58 | 4,54 |
| | 3,29 | 3,46 | 3,65 | 3,67 | 2,89 | 4,35 | 7,62 | 4,31 | 4,33 |
| Moyenne ($\mu$mol/g) %STD | 3,34 3% | 3,45 1% | 3,56 3% | 3,57 3% | 2,97 3% | 4,21 3% | **7,70** 1% | 4,34 5% | 4,43 2% |

Les calculs d'analyse statistique des concentrations en phénol sont donnés dans le tableau 5 suivant.

**Tableau 5**
**Analyse statistique des concentrations en Phénol**

| N°Ech. | 1 | 2 | 3 | 4 | 5 | 6 | 7 | 8 |
|---|---|---|---|---|---|---|---|---|
| 2 | non | / | / | / | / | / | / | / |
| 3 | non | non | / | / | / | / | / | / |
| 4 | non | non | non | / | / | / | / | / |
| 5 | oui | oui | oui | oui | / | / | / | / |
| 6 | oui | oui | oui | oui | oui | / | / | / |
| 7 | oui | oui | oui | oui | oui | oui | / | / |
| 8 | oui | oui | oui | oui | oui | non | oui | / |
| 9 | oui | oui | oui | oui | oui | non | oui | non |

Les mentions « oui » et « non » ont les significations données plus haut.

Cette méthode met à nouveau en évidence que l'échantillon Lalmin Antiox. (échantillon de levure n°7) présente un pouvoir antioxydant qui est très significativement supérieur à celui des échantillons ayant subi des traitements différents. En effet, il apparaît que l'addition :

- du GSH inorganique à une levure classique (1 vs. 3) ne permet pas d'augmenter significativement le pouvoir antioxydant (3,34 / 3,56).
- du GSH organique à une levure classique (1 vs. 8) permet d'augmenter significativement le pouvoir antioxydant (3,34 / 4,34).
- du GSH inorganique à une levure riche en Se organique (5 vs. 6) permet d'augmenter significativement le pouvoir antioxydant (2,97 vs. 4,21).
- du GSH inorganique à une levure riche en Se inorganique (2 vs. 4) ne permet pas d'augmenter significativement le pouvoir antioxydant (3,45 vs. 3,57).
- du Se inorganique (1 vs. 2) ou organique (1 vs. 5) à une levure classique ne permet pas d'augmenter le pouvoir antioxydant (3,34 / 3,45 / 2,97).
- du Se inorganique à une levure riche en GSH organique (8 vs. 9) ne permet pas d'augmenter le pouvoir antioxydant (4,34 / 4,43).
- du Se inorganique à une levure riche en GSH inorganique (3 vs. 4) ne permet pas d'augmenter le pouvoir antioxydant (3,56 / 3,57).

En revanche, en combinant l'apport de Se et GSH organique dans l'échantillon de Lalmin Antiox. (échantillon 7), on augmente très significativement le pouvoir antioxydant vs. les autres traitements (7,70).

Cet échantillon de levure présente un pouvoir antioxydant qui est très significativement supérieur à celui observé en appliquant d'autres traitements aux levures et confirme l'intérêt de l'apport simultané par une même levure de Se organique et de GSH organique ajoutés en cours de fermentation, pour améliorer les effets anti-oxydants du glutathion.

**c- Méthode LAG-TIME / LAG-TIME lié**

*Préparation des échantillons*

On prépare 1mM d'échantillon en mélangeant 395 mg d'échantillon de base à 10 mL d'eau distillée. On soumet le mélange à agitation au Vortex pendant 5 minutes, puis à une centrifugation pendant 10 minutes. On récupère 1mM de surnageant. Ce produit est prêt à l'emploi. (L'échantillon n°7 a été broyé en une poudre fine avant d'être mélangé à l'eau).

*Préparation LDL/VLDL*

On opère comme décrit dans Methods of Enzymology, Vol 335, p103-114, 2001.

*Matériel et Méthode*

La procédure décrite dans la publication J. Agric. Food Chem. 1995, 43:2798-2799 a été utilisée pour mesurer la vitesse d'oxydation des LDL seuls (témoin) et des LDL auxquels on a ajouté un antioxydant. Cette expérimentation permet ainsi de mesurer la capacité de l'antioxydant testé à inhiber l'oxydation.

La préparation de LDL/VLDL et les différents échantillons ont été dilués dans du PBS afin d'obtenir la concentration souhaitée (70 $\mu$g/mL de LDL/VLDL). Le lot témoin contenait seulement la préparation de LDL/VLDL à 70 $\mu$g/mL et le PBS. L'absorbance du PBS, du lot témoin et des autres échantillons a été effectuée à une longueur d'onde de 234 nm pendant 10 min sur un appareil Genesys 5. Ensuite 25 $\mu$M de $Cu^{2+}$ (concentration finale dans la solution) ont été ajoutés mélangés et la mesure d'absorbance a été effectuée toutes les 5 minutes pendant 6 à 8 heures. Les résultats obtenus sont donnés dans le tableau 6 ci-après.

**Tableau 6**
**Inhibition de 1$\mu$M d'échantillon**

| N°Ech. | Témoin | #5 non-lié | #7 non-lié | Sélénométhionine non-liée (Se-Me) | Tocophérol non-lié |
|---|---|---|---|---|---|
| Lag Time (secondes) | 3035 | 9480 | 25371 | 2805 | 7285 |
| Augmentation du Lag Time 1 (%) | | 212% | 736% | -8% | 140% |

$$\text{Augmentation du Lag Time 1} = \frac{\text{Lag Time de l'Echantillon} - \text{Lag Time du Témoin}}{\text{Lag Time du Témoin}} \times 100$$

Les calculs d'analyse statistique des concentrations en tocophérol sont rapportés dans le tableau 7.

**Tableau 7**
**Analyse statistique de l'inhibition de 1$\mu$M d'échantillon**

| N°Ech. | Témoin | #5 | #7 | Se-Me | Tocophérol |
|---|---|---|---|---|---|
| #5 | oui | / | / | / | / |
| #7 | oui | oui | / | / | / |
| Se-Me | non | oui | oui | / | / |
| Tocophérol | oui | oui | oui | oui | / |
| Les mentions « oui » et « non » ont les significations données plus haut. | | | | | |

Comparativement au témoin, l'échantillon de Se-Me n'a pas montré de signes d'inhibition à cette faible concentration. Cependant, le tocophérol et les échantillons 5 & 7 ont montré des niveaux élevés d'inhibition. Ces échantillons présentent une inhibition significativement supérieure au tocophérol. Le pouvoir antioxydant de ces échantillons est supérieur à celui de la Sélénométhionine. L'échantillon 7 de Lalmin Antiox. présente une inhibition significativement supérieure à celle de l'échantillon 5.

## LAG TIME Lié

### Matériel et Méthode

Les LDL et les différents antioxydants sont inoculés afin que des liaisons se créent. Ce phénomène est censé représenter ce qui se passe dans le plasma lorsqu'un antioxydant ayant la capacité de se lier est absorbé.

Les différents échantillons et la préparation de LDL/VLDL ont été dilués à 1 μM et 70 μg/mL respectivement dans du PBS, puis incubés à température ambiante pendant 2 heures. Ensuite 25 μM de $Cu^{2+}$ (concentration finale dans la solution) ont été ajoutés, mélangés et la mesure d'absorbance a été effectuée toutes les 5 minutes pendant 6 à 8 heures. L'absorbance des échantillons a été mesurée à une longueur d'onde de 234 nm pendant 10 min sur un appareil Genesys 5. Le tocophérol a été utilisé comme témoin positif étant donné qu'il est connu pour se lier au LDL qui est généralement un transporteur du tocophérol dans le plasma.

On rapporte les résultats obtenus dans le tableau 8 ci-après.

**Tableau 8**
**Inhibition de 1μM d'échantillon**

| N°Ech. | Témoin | #5 lié | #7 lié | Sélénométhionine liée | Tocophérol lié |
|---|---|---|---|---|---|
| Lag Time (secondes) | 3035 | 21867 | / | 6267 | 20940 |
| Augmentation du Lag Time 1 (%) | 620% | / | | 106% | 590% |
| Augmentation du Lag Time 2(%) | 131% | / | | 123% | 187% |

$$\text{Augmentation du Lag Time 1} = \frac{\text{Lag Time de l'Echantillon - Lag Time du Témoin}}{\text{Lag Time du Témoin}} \times 100$$

$$\text{Augmentation du Lag Time 2} = \frac{\text{Lag Time de l'Ech.lié - Lag Time de l'Ech. non-lié}}{\text{Lag Time de l'Ech. non-lié}} \times 100$$

Les résultats de l'analyse statistique de l'inhibition de 1μM d'échantillon sont donnés dans le tableau 9 ci-dessous.

**Tableau 9**

| N°Ech. | Témoin | #5 | #7 | Se-Me | Tocophérol | #5 lié | Se-Me lié |
|---|---|---|---|---|---|---|---|
| #5 | oui | / | / | / | / | / | / |
| #7 | oui | oui | / | / | / | / | / |
| Se-Me | non | oui | oui | / | / | / | / |
| Tocophérol | oui | oui | oui | oui | / | / | / |
| #5 lié | oui | oui | oui | oui | oui | / | / |
| Se-Me lié | oui | oui | oui | oui | non | oui | / |
| Tocophérol lié | oui | oui | oui | oui | oui | oui | oui |

Les mentions « oui » et « non » ont les significations données plus haut.

Comparativement à l'expérimentation précédente, les LAG TIME de tous les échantillons testés ont augmenté de plus de 100% (celui du Lalmin Antiox. n'est pas représenté car >10 heures).

Ces 2 expérimentations montrent que l'échantillon 7 de Lalmin Antiox. présente un pouvoir antioxydant beaucoup plus important que l'échantillon 5. L'échantillon 7 non-lié est même supérieur à l'échantillon 5 lié. L'échantillon 7 lié présente un LAG TIME de plus de 10 heures, soit 36 000 secondes, ce qui est 50% au-dessus du tocophérol lié et de l'échantillon 5 lié.

## LAG TIME Lié

<u>Exemple 2 :</u> Comparaison de l'activité antioxydante d'échantillons en UV-Visible

On rapporte ci-après les résultats obtenus en comparant les propriétés antioxydants de deux échantillons, dont un selon l'invention, vis-à-vis de leur capacité à réduire le radical 2,2-diphényl-1-picryl-hydrazyl (DPPH•) (UV- Visible).

Le 2,2-diphényl-1-picryl-hydrazyl (DPPH•) est un radical organique stable présentant une absorption caractéristique à $\lambda$=523 nm dans un mélange MeOH/$H_2$O (2/1). Le DPPH• présente une couleur violette intense et ce composé reste stable, sous forme solide, pendant des années. Un antioxydant, donneur de proton, est capable de réduire ce radical (DPPH•→ DPPH2), ce qui entraîne une décroissance de son absorbance. La capacité antioxydante des composés est ainsi déterminée en évaluant le pourcentage d'inhibition de l'absorbance du DPPH• à 523 nm.

La concentration massique des extraits diminuant l'absorbance de DPPH• à 523 nm de 50% ($CI_{50}$) est déterminée à l'aide de l'équation de la droite : Absorbance du DPPH• à 523 nm = f(concentration du mélange testé). L'extrait possédant la valeur de $CI_{50}$ la plus faible est l'extrait possédant la capacité antioxydante la plus efficace.

On utilise le DPPH• commercialisé par Aldrich et la vitamine C commercialisée par Merk. On rapporte les résultats obtenus avec les échantillons E1 et E2 suivants :

E1 : Lalmin Antiox - Levure enrichie concomitamment en Sélénium et en Glutathion (Se : 2000 ppm ; GSH : 20 mg.g$^{-1}$).

E2 :FSR - Levure enrichie en Glutathion + LalminSe - Levure enrichie en Sélénium

Dans ces essais, les concentrations en sélénium et en glutathion sont telles que la solution de E2 contient autant de sélénium et de glutathion que celle de E1. La méthode mise en oeuvre permet d'évaluer le pouvoir antioxydant global du mélange et de le comparer à celui de substances de référence dans les mêmes conditions expérimentales. L'activité antioxydante est évaluée en mesurant la capacité de l'extrait à piéger le radical DPPH•.

Les résultats obtenus sont donnés dans les tableaux 10 et 11 ci-dessous :

**Tableau 10**

**$CI_{50}$ des molécules de référence, glutathion et vitamine C (n=3).**

| temps (min) | moyenne | Ecart type |
|---|---|---|
| | $CI_{50}$ (mg.L$^{-1}$) | |
| **Glutathion** | | |
| 10 | 292,97 | 13,90 |
| 20 | 233,84 | 9,73 |
| 30 | 202,13 | 8,31 |
| **vitamine C** | | |
| 10 | 5,24 | 0,27 |
| 20 | 5,20 | 0,25 |
| 30 | 5,16 | 0,30 |

**Tableau 11**

**$CI_{50}$ des échantillons E1 et E2**

| Echantillon | $CI_{50}$ (mg.L$^{-1}$) par rapport à la masse de poudre pesée |
|---|---|
| E1 | 1461 |
| E2 | 6944 |

L'examen des résultats obtenus montre que :

➢ L'activité antioxydante dans cet essai est plus élevée pour la vitamine C que pour le glutathion.
➢ La $CI_{50}$ du glutathion est égale à 202,13 mg.L$^{-1}$ soit 40 fois plus élevée que pour celle de la vitamine C (tableau 10).
➢ Les CI50 de E1 et de E2 sont respectivement de 1461 mg.L$^{-1}$ et de 6944 mg.L$^{-1}$, montrant la supériorité de l'effet antioxydant pour les levures enrichies concomitamment en sélénium et en glutathion.

Exemple 3 : Utilisation des préparations de levures dans l'alimentation

On utilise une levure enrichie simultanément en glutathion et en sélénium, de manière à amener le pouvoir réducteur du mélange à plus de 15 $\mu$mole de phénol/g de poudre selon la méthode FOLIN ou à plus de 5 $\mu$mole/g selon le test de FRAP.

La préparation de levure est broyée pour obtenir une poudre. On incorpore la préparation de levure sous forme pulvérulente dans une pâte à biscuits.

**Revendications**

1. Préparations de levures, présentant des propriétés anti-oxydantes améliorées, **caractérisées en ce qu'**elles comprennent des levures enrichies concomitamment en glutathion organique endogène, produit par la levure même durant la fermentation et en sélénium organique, ce sélénium étant ajouté au milieu de fermentation et incorporé par la levure durant la fermentation, à raison d'au moins 15 mg/g de matières sèches en ce qui concerne le glutathion et à raison d'au moins 500 ppm, avantageusement de l'ordre de 2000 ppm, en ce qui concerne le sélénium.

2. Préparations de levures selon la revendication 1, **caractérisées en ce que** lesdites levures sont des levures du genre *Saccharomyces* et notamment de l'espèce *cerevisiae.*

3. Préparations de levures selon la revendication 2, **caractérisées en ce qu'**il s'agit de levures de panification.

4. Utilisation de préparations de levures selon l'une quelconque des revendications 1 à 3, comme additifs anti-oxydants.

5. Utilisation selon la revendication 4, dans les aliments ou nutriments destinés à l'homme ou l'animal.

6. Utilisation de préparations de levures selon l'une quelconque des revendications 1 à 3, pour préparer des alicaments destinés à l'homme ou l'animal.

7. Utilisation selon la revendication 4, dans le domaine cosmétique.

**Patentansprüche**

1. Hefezubereitungen, die verbesserte oxidationshemmende Eigenschaften aufweisen, **dadurch gekennzeichnet, dass** sie Hefen umfassen, die gleichzeitig angereichert sind mit organischem endogenem Glutathion, das durch die Hefe selbst während der Fermentation erzeugt wird, und mit organischem Selen, wobei dieses Selen dem Fermentationsmedium zugegeben wird und während der Fermentation durch die Hefe aufgenommen wird, in einer Menge von wenigstens 15 mg/g Trockenmasse was das Glutathion betrifft und in einer Menge von wenigstens 500 ppm, vorteilhafterweise in der Größenordnung von 2000 ppm, was das Selen betrifft.

2. Hefezubereitungen nach Anspruch 1, **dadurch gekennzeichnet, dass** die Hefen Hefen von der Gattung *Saccharomyces* und insbesondere der Spezies *cerevisiae* sind.

3. Hefezubereitungen nach Anspruch 2, **dadurch gekennzeichnet, dass** es sich um Backhefen handelt.

4. Verwendung von Hefezubereitungen nach einem der Ansprüche 1 bis 3, als oxidationshemmende Zusätze.

5. Verwendung nach Anspruch 4, in Nahrungsmitteln oder Nährstoffen, die für Mensch oder Tier bestimmt sind.

6. Verwendung von Hefezubereitungen nach einem der Ansprüche 1 bis 3, für die Herstellung von für Mensch oder Tier bestimmten Nutrazeutika.

7. Verwendung nach Anspruch 4, im Kosmetikbereich.

**Claims**

1. Yeast preparations, having improved antioxidant properties, **characterized in that** they comprise yeasts enriched concomitantly with endogenous glutathione produced by the yeast even during fermentation and with organic selenium, said selenium being added to the fermemtation medium and incorporated by the yeast during said fermentation, in an amount of at least 15 mg/g of dry matter for glutathione and in an amount of at least 500 ppm, advantageously of around 2000 ppm for selenium.

2. The yeast preparations as claimed in claim 1, **characterized in that** said yeasts are yeasts of the *Saccharomyces* genus and especially of the *cerevisiae* species.

3. The yeast preparations as claimed in claim 2, **characterized in that** they are breadmaking yeasts.

4. The use of yeast preparations as claimed in any one of claims 1 to 3, as antioxidant additives.

5. The use as claimed in claim 4, in foods or nutrients intended for humans or animals.

6. The use of yeast preparations as claimed in any one of claims 1 to 3, for preparing functional foods for humans or animals.

7. The use as claimed in claim 4, in the cosmetics field.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 4530846 A
- JP 52156994 B
- JP 61132282 B

**Littérature non-brevet citée dans la description**

- *Methods of Enzymology,* 2001, vol. 335, 103-114
- *Anal.Biochem.,* 15 Juillet 1996, vol. 239 (1), 70-6
- *Free Radic.Biol.Med.,* 15 Juin 2000, vol. 28 (12), 1815-26
- *J. Agric. Food Chem.,* 1995, vol. 43, 2798-2799